# EUROPEAN PATENT SPECIFICATION

(11) **EP 1 214 334 B1**
(45) Date of publication and mention of the grant of the patent: **11.10.2006**
(21) Application number: 00939675.5
(22) Date of filing: 08.06.2000
(51) Int. Cl.: C12N 15/53, C12N 9/02, C12N 15/82, C12N 15/10, C12N 5/10, A01H 5/00

(54) **MUTANT FATTY ACID DESATURASE**
MUTIERTE FETTSÄUREDESATURASE
DESATURASE MUTANTE D'ACIDES GRAS

(30) Priority: 09.06.1999 US 328550
(43) Date of publication of application: 19.06.2002
(73) Proprietor: Brookhaven Science Associates LLC, Upton, New York 11973-5000 (US)
(72) Inventor: SHANKLIN, John, Shoreham, NY 11786 (US)
(74) Representative: Kirkham, Nicholas Andrew
(86) International application number: PCT/US2000/015741
(87) International publication number: WO 2000/075170

(56) References cited:
- WO-A-98/06735
- US-A- 5 614 400
- US-A- 5 856 157
- US-A- 5 888 790
- CAHOON ET AL: "Redesign of soluble fatty acid desaturases from plants for altered substrate specificity and double bond position" PROCEEDINGS OF THE NATIONAL ACADEMY OF SCIENCES OF USA, NATIONAL ACADEMY OF SCIENCE. WASHINGTON, US, vol. 94, May 1997 (1997-05), pages 4872-4877, XP002124103 ISSN: 0027-8424
- CAHOON E B ET AL: "APPROACHES TO THE DESIGN OF ACYL-ACP DESATURASES WITH ALTERED FATTY ACID CHAIN-LENGTH AND DOUBLE BOND POSITIONAL SPECIFITIES" PHYSIOLOGY, BIOCHEMISTRY AND MOLECULAR BIOLOGY OF PLANT LIPIDS, XX, XX, 1997, pages 374-376, XP001017917
- LINDQVIST Y ET AL: "CRYSTAL STRUCTURE OF DELTA9 STEAROYL-ACYL CARRIER PROTEIN DESATURASE FROM CASTOR SEED AND ITS RELATIONSHIP TO OTHER DI-IRON PROTEINS" EMBO JOURNAL, OXFORD UNIVERSITY PRESS, SURREY, GB, vol. 15, no. 16, 1996, pages 4081-4092, XP000941999 ISSN: 0261-4189

## Description

### Background of the Invention

Fatty acid biosynthesis in higher plants has recently attracted increased interest because of the possible use of plant oils as renewable sources for reduced carbon. The diversity of fatty acid forms in wild plants is vast compared to that found in crop plants. This is reflected in their chain length, the number and position of double bonds, and the position and occurrence of a variety of other functional groups.

In plants, fatty acid biosynthesis occurs in the chloroplasts of green tissue or in the plastids of nonphotosynthetic tissues. The primary products in most plants are acyl carrier protein (ACP) esters of the saturated palmitic and stearic acids. Two types of desaturase molecules are involved in the production of unsaturated fatty acids, soluble, and integral membrane proteins. Desaturases are specific for a particular substrate carbon-chain length and introduce the double bond between specific carbon atoms by counting from the carboxyl end of the fatty acid; for instance, the Δ⁹ desaturase is specific for stearoyl-ACP, and introduces a double bond between carbon 9 and 10. The introduction and use of desaturase isoforms with characteristic chain length preferences and double bond positions of these molecules, into agricultural crops offers a way to manipulate the physical properties and commercial uses of conventional plant oils. Unfortunately, the introduction of acyl-ACP desaturase isoforms into non-native host plants has yet to lead to the efficient production of unusual monoenes.

As the genes encoding more desaturase enzymes are identified it is becoming apparent that many of the different activities are derived from relatively few common archetypes encoding soluble and membrane classes of desaturases. Molecular modeling of soluble acyl-ACP desaturases has identified amino acid residues within the substrate binding channel which are in very close proximity to the fatty acid substrate. Such residues are referred to as "contact residues". Previous research has shown that the modification of a contact residue (and in some cases, other residues) can alter the chain-length and double bond positional specificities of an acyl-ACP desaturase. The ability co manipulate the chain length preferences and double bond positions of desaturases has great potencial wich regard to generation and use of modified desaturases in the production of commercially useful products, such as vegetable oils rich in monounsaturated fatty acids. Such vegetable oils are important in human nutrition and can be used as renewable sources of industrial chemicals.

### Summary of the Invention

The present invention relates to a mutant castor Δ⁹ - 18 : 0 - ACP desaturase having each of the substitutions:
a) Ala for Met at residue 114;
b) Arg for Thr ac residue 117;
c) Gly for Leu at residue 118;
d) Val for Pro at residue 179;
e) Val for Thr at residue 181; and
f) Leu for Gly at residue 188.

In another aspect the invention relates to a DNA expression construct comprising, in expressible form, a nucleic acid sequence which encodes a mutant castor Δ⁹ - 18 : 0 - ACP desaturase having each of the amino acid substitutions:
a) Ala for Met at residue 114;
b) Arg for Thr at residue 117;
c) Gly for Leu at residue 118;
d) Val for Pro at residue 179;
e) Val for Thr at residue 181; and
f) Leu for Gly at residue 188.

In another aspect the invention relates co a cell transformed with a DNA expression construct comprising, in expressible form, a nucleic acid sequence which encodes a mutant castor Δ⁹ - 18 : 0 - ACP desaturase having each of the substitutions:
a) Ala for Met at residue 114;
b) Arg for Thr at residue 117;
c) Gly for Leu at residue 118;
d) Val for Pro at residue 179;
e) Val for Thr at residue 181; and
f) Leu for Gly at residue 188.
Preferred features of the cell are defined in claim 5.

In another aspect the invention relates to a transgenic plant expressing a nucleic acid sequence which encodes a mutant castor Δ⁹ - 18 : 0 - ACP desaturase having each of the amino acid substitutions:
a) Ala for Met at residue 114;
b) Arg for Thr at residue 117;
c) Gly for Leu at residue 118;
d) Val for Pro at residue 179;
e) Val for Thr at residue 181; and
f) Leu for Gly at residue 188.
A preferred feature of the transgenic plant is defined in claim 6.

A method for producing a mutant of a fatty acid desaturase is also described in which the original desaturase has 18-carbon chain substrate specificity, and the mutant produced has substantially increased activity towards fatty acid substrates with chains containing fewer than 18 carbons, relative to the original desacurase. The method involves inducing one or more mutations in the nucleic acid sequence encoding the original desaturase, transforming the mutated nucleic acid sequence into a cell which is a unsaturated fatty acid auxotroph, and selecting for recipient cells which have received a mutant fatty acid desaturase with reduced carbon chain length substrate specificity. The mutated nucleic acid sequences may be transformed into MH13 *E. coli,* which are then grown in the absence of exogenous unsaturated fatty acids to select for recipient MH13 cells which express the desired mutants.

Mutants of castor Δ⁹ 18:0-ACP desaturase arise from amino acid substitutions at specific residues. These mutants have altered substrate specificities, of 16- or fewer carbon chains. The expression of the mutant desaturase molecules in individual cells and also in transgenic plants. for the production of specific fatty acid products is described herein.

A method for specifically altering a function of a protein through directed mutagenesis is also described. The method involves identifying candidate amino acid positions of the protein which, when mutated, are predicted to alter the function. A library of mutants of the protein which are produced by randomization of the amino acid at each candidate position, in combination with randomization of every other candidate position is generated, and mutants which exhibit the desired specific alteration of function are identified from the library. Candidate amino acid positions are identified by a combination of methods. some examples being random mutagenesis. Structural analysis of the protein, and sequence analysis of the protein. Examples of functions which the method can be used to alter include enzymatic functions, binding functions, and structural functions. The method described herein is compared to the method of random mutagenesis in alteration of castor Δ⁹ 18:0-ACP desacurase substrate specificity.

### Brief Description of the Drawings

Figure 1 lists the amino acid sequence of mature castor enzyme, and the corresonding nucleic acid coding sequence.
Figure 2 is a table which lists five mutants produced by combinatorial positional randomization and their respective activities with 14, 16 and 18 carbon substrates.
Figure 3 is a table comparing a mutant produced by combinatorial positional randomization co mutants obtained by single position randomization and mutants obtained by random mutagenesis.
Figure 4 is a diagram illustrating the primers used for full positional randomization of site 117 of castor Δ⁹ 18:0-ACP desaturase.
Figure 5 is a diagram illustrating the primers used for the generation of a combinatorial library of castor Δ⁹ 18:0-ACP desaturase with full positional randomization of positions 114, 117, 118, 179, 181, and 188.

### Detailed Description of the Invention

The use of a bacterial selection system for the selection of mutant desaturase molecules which have 18-carbon chain length substrate specificities prior co the introduction of the mutation is described herein. The identified mutants have mutations which reduce the chain length substrate specificity of the desaturase molecule. A preferred bacterial strain used in the selection system, *E. coli* MH13. is an unsaturated fatty acid auxotroph- MH13 requires exogenous unsaturated fatty acids to proliferate. Introduction cf a functional desacurase enzyme which has subscancial activity towards fatty acid substrates with chains containing 16 or fewer carbons complement this auxotrophy. allowing for the growth and proliferation of the bacteria in che absence of exogenous unsaturated fatty acids. Desaturase enzymes which specifically utilize 18-carbon chain length substrates cannot complement che auxotrophy due to the low levels of such substrates in the bacteria. This observation has been exploiced as a selection system for identifying mutants of an 18-carbon specific fatty acid desaturase which can utilize 16-carbon or 14-carbon substrates. While *E. coli* MH13 is a preferred host cell, one of skill in the arc will recognize that other host cell types may be employed.

A method of producing a mutant of a fatty acid desaturase, the mutant being characterized as having a reduced carbon-chain length substrate specificity from the original fatty acid desaturase, is described herein. The method requires nucleic acid sequences encoding the fatty acid desaturase with 18-carbon chain length substrate specificity. To produce the mutant, mutations are induced in the nucleic acid sequence encoding the fatty acid desaturase. The mutated nucleic acid sequence is then transformed into the MH13 *E. coli* cells under conditions appropriate for expression of the mutated sequence. The transformed MH13 *E. coli* cells are then selected for the ability to grow in the absence of supplemented reduced fatty acids. Survival of a transformed MH13 *E. coli* indicates the acquisition of a mutant fatty acid desaturase which complements the fatty acid auxotrophy of MH13.

A mutant fatty acid desaturase identified by the above selection assay has a substantial increase in the activity cowards fatty acid substrates with chains containing fewer than 18 carbons, relative to the original desaturase. A substantial increase in substrate specificity with respect to the original desaturase is one that produces the accumulation of unsaturated fatty acids which result from a desaturation by the mutant desaturase within the host organism- Substantial increase in activity is defined to be at least three fold higher than that of the non-mutagenized precursor desaturase. The increase in activity of the mutant desaturase is at least ten fold higher than the nonmucagenized precursor desaturase.

The method is suitable for producing mutants of any fatty acid desaturase with 18-carbon chain substrate specificity, for which a nucleic acid sequence is available. Expression of the nucleic acid sequence should result in the production of a mature fatty acid desaturase. The nucleic acid sequences may correspond to wild type sequences or may have silent mutations which do not affect the amino acid sequence of the transcribed product. Alternatively. the nucleic acid sequences used may encode a functional fatty acid desaturase, whose amino acid sequence varies from wild type, for example with conservative amino acid substitutions that do not effect function in regard to carbon chain length substrate specificity. Such a mutant desaturase may be desirable when incorporating several different functional mutations into one mutant.

The fatty acid desaturase is a plant fatty acid desacurase. There are two types of plant fatty acid desaturases, soluble (acyl-ACP desaturases). and integral membrane (acyl lipid desaturases). Previous molecular modeling and structure-function studies have characterized regions involved in substrate specificity of acyl-ACP desaturases, as described in Cahoon ec al., U.S. Patent 5.705,391 (1998). When expressed in *E. coli,* variant desaturase isoforms (e.g. isoforms thought responsible for the accumulation of unusual fatty acids in species that accumulate monoenes other than 18:1 Δ⁹) show poor accumulation, are unstable, and have lower specific activities compared to Δ⁹ 18:0-ACP desacurases. The reasons for this are not yet fully understood, but recent results suggest the presence of "specificity factors" which may potentiate the activity of a specific acyl-ACP desaturase. For these reasons, Δ⁹ 18:0-ACP desaturases are best suited for *in vitro* experimentation utilizing *E*. *coli.*

The MH13 *E*. *coli* may have exogenous plant ferredoxin. This can be accomplished by introduction of an expression vector containing sequences which encode plant-type ferredoxin (e.g. *Arabaena* vegetative ferredoxin), and the application of selective pressure to the resulting bacteria. The presence of plant-type ferredoxin, the redox partner of the plant desaturases. facilitates the function of the plant desacurase in *E. coli.* The presence of plant-type ferredoxin in the selection system allows for the selection of mutants with lower specific activities, whereas mutants which complement MH13 in the absence of plant-type ferredoxin are expected to have comparatively higher specific activities. The selection system described above is most appropriate for use in selecting mutants with the desired substrate specificity from a population of heterogenous mutant fatty acid desaturase molecules. By transforming a population of mutated nucleic acid sequences, entire libraries of mutants can be screened for the ability to complement the MH13 auxotrophy.

Any type of mutation which has the potential to result in a modified fatty acid desaturase protein product can be induced in the nucleic acid sequences. Logic based approaches of introducing amino acid substitutions into residues which interact wich substrate are sound but can be very labor intensive; and are only suited to cases in which structural information is available. Such methods have been successfully employed for chain length for the soluble desaturases, and for the introduction of double-bond versus hydroxyl group for the membrane class of enzymes (Cahoon et al., *Proc Natl Acad Sci USA 94:* 4872-4877 (1997); Shanklin et al., *Annu. Rev. Plant Physiol. Plant Mol Biol. 49*: 611-641 (1998)). Experiments described in the Exemplification section which follows demonstrate a form of site directed mutagenesis which specifically target a particular codon. or codons, to produce amino acid randomization at specific position(s) within the protein product. These experiments also describe utilization of random mutagenesis. which has the potential co identify additional amino acids involved in substrate specificity. The use of random mutagenesis is perhaps the most powerful method because it does noc rely on assumptions about which residues are important. The present method has the ability to identify substitution mutations at positions which are amino acid contact residues (positions which are located nearest the substrate within the binding channel) and also positions which affect the substrate specificity without directly contacting the substrate (Cahoon et al., *Proc Natl Acad Sci USA 94*: 4872-4877 (1997)).

Once mutated, the nucleic acid sequences are transformed into the MH13 cells. Transformation is preferably accomplished by electroporation, but alternative methods known to one of skill in the art can also be used. Following transformation, the cells are selected for the presence of the mutant fatty acid desaturase. This is accomplished by growth on selective media (e.g. media lacking exogenously supplied unsaturated fatty acids). The media can be either solid or liquid. Using several rounds of selection, and/or varying or augmenting the selective pressures involved is also useful in increasing the number of mutants identified by the method.

The engineering of desaturase proteins with characteristic substrate chain length preferences is also described herein. Such isoforms, when introduced into cells or organisms (e.g. agricultural crops) can be used co manipulate the physical properties and commercial uses of conventional plant oils. Cells and organisms which express these engineered desaturases are useful in the production of commercially useful products, such as vegetable oils rich in monounsaturated fatty acids, which have many potential uses, for example in human nutrition or as industrial chemicals.

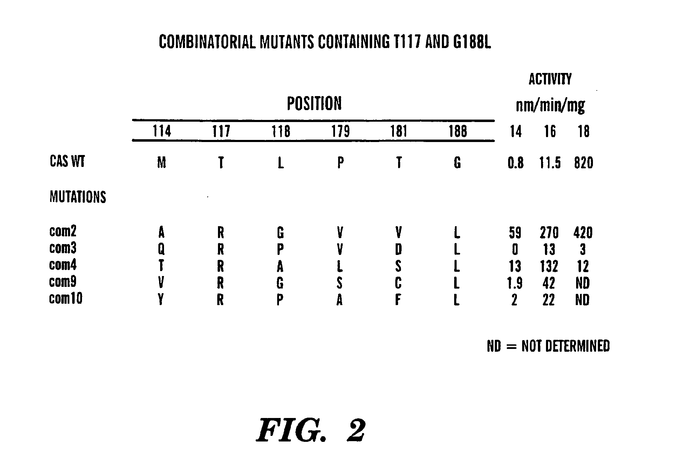

The Exemplification section below details experiments where the above described method was used to identify mutants of castor Δ⁹-18:0-ACP desaturase which have modified substrate specificities. Mutations in the coding region which result in amino acid substitutions at position 114, 117, 118, 179, 181, or 188, and combined substitutions at positions 114 and 188, are described, along with the resulting altered specificity of these mutant proteins as compared to wild type. Mutations in the coding region of castor Δ⁹-18:0-ACP desaturase which produce a combination of amino acid substitutions at all six positions were also identified, the mutant proteins encoded being referred to as com2, com3, com4, com9, and com10. Figure 2 lists the amino acid substitutions and the specific activities of these mutant proteins for the different substrates.

All five mutant proteins listed in Figure 2 have the amino acid substitutions T117R and G188L, in combination with various substitutions at the remaining four positions. The fact that these two mutations are the optimal changes at their respective positions for reducing chain length specificity suggests that they are likely the primary determinants of the altered specificity in com2. The observation that several other mutants containing this pair of mutations have lower specific activity suggests that the combination of mutations at the remaining four randomized sites can also affect the specific activity of the mutants. This conservation suggests that the substitutions T117R and G118L are responsible for the change in substrate specificity of the five mutants in Figure 2. A mutant desaturase with the combination of T117R and G118L substitutions is expected to have enhanced activity for one or more substrates with 16 or fewer carbons.

A mutant castor Δ⁹-18:0-ACP desaturase which has a subset of the amino acid substitutions of a mutant protein listed in Figure 2 is also described. Such a mutant is expected to also have altered activity towards the different substrates. The mutant castor Δ⁹-18:0-ACP desacurase proteins have between 1 and 6 of the amino acid substitutions of the com2 mutant, in any possible combination. The combination may include at least three of the amino acid substitutions of the com2 mutant. Preferably two of these amino acid substitutions are T117R and G188L. In addition, the mutant Δ⁹-18:0-ACP desaturase proteins may have between 1 and 6 of the amino acid substitutions of the com3 mutant, the com4 mutant, the com9 mutant, or the com10 mutant, respectively, in any possible combination. Alternatively the combination may include at least three of the amino acid substitutions, of the mutant. Preferably two of these substitutions are T117R and G188L. Also described are mutants which have the above lisced amino acid substitutions, and combinations thereof, in combination with any ocher amino acid substitutions, insertions or deletions. These additional substitutions, insertions or deletion, may be silent (e.g. do not affect function of the enzyme) or may further alter enzyme function.

The above listed amino acid substitutions made at the analogous positions in ocher ACP-desaturases. especially in 18:0-ACP desaturases, and preferably in Δ⁹ 18:0-ACP desaturases, are predicted to have the analogous effects on substrate specificity in these proteins as in the disclosed desaturase mutants.

Nucleic acid sequences which encode the mutant proteins described above, can be inserted into a DNA expression vector, which can then be used co express the mutant proteins in cells. Expression vectors which function in either or both prokaryotic and eukaryotic cells exist and are known to chose of skill in the art. The appropriate expression vectors are introduced into either prokaryotic cells, (e.g. bacteria) or eukaryotic cells (e.g. animal cells or plant cells) under conditions appropriate for expression of their coding sequences. Plant cells which express the mutant proteins can be used to produce transgenic plants which express the mutant proteins, and which produce the corresponding fatty acid products of the desaturases.

A method for specifically altering a function of a protein through directed mutagenesis is also described herein. Upon determination of the specific function which is co be altered, candidate amino acid positions of the protein which are predicted to alter the function when mutated, are identified. Several methods for identifying candidate positions are described below. A library of mutants of the protein are generated by randomization of the amino acid encoded at each candidate position, in combination with randomization of every other candidate position within each mutant. This is generally accomplished by generating a library of mutant nucleic acid sequences which encode the mutant proteins through simultaneous randomization of the codons for each candidate position, in combination with randomization of every other candidate position within each mutant. Mutant proteins. encoded by the mutated nucleic acid sequences, which exhibit the desired alteration of function are then identified from the library.

A wide variety of functions are performed by proteins. Some proteins function as enzymes which catalyze reactions (e.g. catabolic, anabolic), some proteins function as binding proteins (e.g. ligand binding receptors, antibodies, adapter proteins), some proteins function as structural proteins (e.g. extracellular matrix proteins). Described herein is a method useful for altering any given function of any given protein. Because many proteins have more than one function, the specific function which is co be altered must first be determined. Often the functions of a , multifunctional protein are independent of one another, allowing one function to be altered without affecting the other function(s) of the protein. In other cases, the functions are interlinked or interdependent, making alteration of a single function more complex. Alteration of a function is broadly defined herein as including any directed change in the function. Such changes include, without limitation, optimization of a function, (e.g. increasing the specific activity of an enzyme, increasing the binding affinity of a binding protein, increasing the integrity or stability of a protein's structure), redirection a functional property of a protein (e.g. modifying the substrate specificity of an enzyme, modifying the binding specificity of a binding protein, modifying a structural component of a protein), and reduction (e.g. abolishing) of a function. Complete alteration of a designated function may necessarily be achieved in stages through sequential alteration of individual components of the function, producing a series of intermediate mutants, the entire process culminating in the generation of a final optimal mutant. Therefore, the process of altering a function of a protein as described herein, is intended to include optimizing, redirecting, or reducing a function of a previously altered protein.

Candidate positions include the positions of amino acids of the wild type protein which are involved (either directly or indirectly) in the function. Importantly, an indication of involvement in the function is all that is required for selection of candidate positions. The direction an individual mutation has or is expected to have on the function is unimportant in the identification of candidate positions. For instance, residues which when mutated individually, result in a decrease of the function, may be identified as candidates. Examples of amino acids which are directly involved with function include, without limitation, residues which make contact with other molecules involved in the function (e.g. substrate or ligand), and also residues which line or define binding sites. Examples of amino acids which have indirect involvement include, without limitation, residues which influence those directly involved residues, such as residues adjacent or near directly involved residues. Proximity need not be limited to primary sequence, but may be from secondary or tertiary structure relationships. In addition, residues may be located near directly involved residues due to the formation of inter- or intra-molecular complexes. The influence that indirectly involved amino acids can have may be steric effects, chemical effects, or a combination effects. Indirectly involved amino acids also include residues which participate in defining an element of the protein structure which is crucial for the function (e.g. the necessary conformation of a protein).

Candidate positions also include positions of amino acids which are not significantly involved (directly or indirectly) in the function of the wild type protein, but which assume a role (direct or indirect) in function through mutagenesis. The term wild type is used herein to refer to the original sequence of a protein prior to mutagenesis, the term being inclusive of previously altered sequences. Mutagenesis which confers a role in function to a previously uninvolved residue commonly involves the substitution of another amino acid at that particular position. However, a new involvement in function may also be conferred to a position by mutagenesis at another position.

The more information one has regarding the protein, the function of the protein which is to be altered, and the residues which participate in the function, the more productively one can go about altering the function. Candidate amino acid positions of the protein are identified by any number of means. Without limitation, such means include, random mutagenesis of the nucleic acid sequences encoding the protein, structural analysis of the protein, and sequence analysis of the protein, often coupled with comparison to related proteins. Methods for identification of candidate positions may be performed with the naturally occurring protein, or alternatively with a mutant version of the protein. In addition, analysis of related proteins (e.g. sequence analysis, structural analysis, mutagenesis) may indicate analogous candidate positions within the protein of interest which are likely involved in the function to be altered. The term related proteins as used herein includes different isomers of a protein, different phenotypes of a protein (e.g. naturally occurring mutants of the same protein) , and any other proteins or fragments thereof which have significant homology to the protein whose specific function is to be altered.

Random mutagenesis coupled with screening for loss of function mutants can identify amino acid positions which are crucial for function of the wild type protein. Random mutagenesis coupled with screening for gain or enhancement of function mutants can identify these crucial positions as well as positions which only minimally participate in wild type function, but have gained an increased role through mutagenesis.

Structural analysis of a protein is also a very powerful tool with which to identify candidate residues-Structural information can be obtained from X-ray crystallography, or from other methods such as nuclear magnetic resonance. Often the structure of a protein performing or the function (e.g. an enzyme bound to substrate or an inhibitor, or a binding protein bound to ligand) provides a significant amount of information regarding the amino acid positions involved in the function.

Preferably. a combination of methods are employed to identify candidate amino acid positions. Preferably, all available means are employed to ensure identification of as many candidate positions as possible.

Once the candidate positions are identified, a library of mutant proteins which have every candidate position within each mutant randomly substituted with 1 of 20 possible amino acids. This method of substituting 1 of the 20 possible amino acids at a specific position within a protein is referred to herein as randomization of the amino acid. Randomization of the amino acid encoded at each and every candidate codon within an individual particular protein is referred co herein as combinatorial full positional randomization. A library of mutant proteins resulting from combinatorial full positional randomization is most easily produced by generating a nucleic acid library of mutated coding sequences of the protein, which have 1 of 20 possible amino acid encoded at every candidate position within. Because this type of mutagenesis allows for the insertion of a codon for the wild type residue, as well as the other 19 residues, at each candidate position, this produces the widest possible variety of mutation combinations. Combinatorial full positional randomization of codons can be accomplished by a variety of methods. One such method is the use of overlap-extension PCR to replace all codons for candidate position amino acids with NNK or NNN. The process of overlap-extension PCR has been used to simultaneously introduce at lease nine independent mutations into a particular coding sequence.

Alternatively, a subset of one or more of the candidate positions are incompletely randomized, while the other candidate positions are fully randomized. That is to say, fewer than the 20 possible amino acids are introduced at one or more designated candidate positions, to more specifically direct the mutagenesis. This is accomplished by randomly replacing the subset of candidate position codons of the nucleic acid sequence which encode the protein, with codons that encode the desired subset of amino acids, while introducing codons which encode all 20 amino acids at the other candidate positions.

Once che library is generated, mutant proteins which exhibit the desired altered function are identified. This is most efficiently accomplished by using a functional selection process. The mutated nucleic acid sequences are expressed individually, preferably by individual introduction into a single celled organism under conditions appropriate for expression. Once expressed, the mutant proteins which exhibit the desired function may be selected by their function (e.g. a complementation assay). Alternatively, populations of mutants generated can be screened for the desired altered function (e.g. by a rapid screening process). Each mutant generated can also be individually assayed for the desired altered function.

Experiments detailed in the Exemplification section which follows were performed to modify the substrate specificity and specific activity of castor Δ⁹-18:0-ACP desaturase. Structural analysis of the protein was combined with random mutagenesis to identify candidate residues.

Random mutagenesis was used to identify candidate residues of the desaturase by a functional assay. Theoretically, this method of identification has the potential to identify residues which may or may not line the substrate binding cavity, they are simply identified by a functional assay, thus this method of identifying residues likely to participate in function is applicable to both enzymes for which a structure is known. and enzymes for which a structure is unknown. All relevant knowledge should be included in compiling the list of candidate amino acid positions to be randomized.

One distinguishing feature of the method described herein is that combinatorial full positional randomization is performed simultaneously on all candidate positions which are identified. Previous approaches to directed mutagenesis co specifically alter a function of a protein have used a multistep approach, where one residue is mutated, the mutant is characterized, and then that mutant is subjected to another round of single position mutagenesis. This standard approach results in each subsequently produced mutant carrying over specific mutations from the last mutant product. Thus, each subsequent mutant identified is necessarily constrained by properties inherited from the mutant from which it is generated, thus limiting the direction the mutagenesis may take to achieve the desired function. By eliminating this limitation, the method described herein generates a wider variety of mutants which demonstrate the desired activity, from which one can select an optimal mutant.

### Exemplification

### SECTION I: PRELIMINARY STUDIES

A mutagenesis and selection approach was employed to identify amino acid substitution mutations in plant fatty acid desaturases which modify substrate specificity. Acyl-ACP desaturases are functionally active when expressed in *E. coli.* Δ⁹-18:0-ACP desaturases are unable to alter the fatty acid profile of *E. coli* due to a lack of appropriate substrate (Thompson et al., *Proc Natl Acad Sci U S A 88:* 2578-2582 (1991); Cahoon et al., *Proc Natl Acad Sci U S A 94:* 4872-4877 (1997)). However, a desaturase with 16:0 specificity was shown to alter the fatty acid profile of *E. coli.* Thus, 18:0 desaturases cannot complement the *E. coli* mutant MH13, an unsaturated fatty acid auxotroph, but desaturases with specificities with 16 or fewer carbons are able to complement this auxotrophy. Thus, the MH13 *E. coli* strain was used to select for mutants of an 18-carbon desaturase which can utilize 16- or 14-carbon substrates in a complementation assay.

To facilitate the function of a plant acyl-ACP desaturase in *E*. *coli,* an expression vector containing the gene for plant-type ferredoxin, the redox partner of the plant desaturase, was transformed into the MH13 *E*. *coli* and maintained under selective pressure. These cells, MH13(pACYC/LacAnFd) were used in the following experiments.

Castor-Δ⁹ 18:0-ACP desaturase was subjected to one of two types of mutagenesis, site directed or random mutagenesis, prior to introduction into the MH13 cells. PCR was used in site directed mutagenesis to randomized a targeted codon corresponding to a specified residue in the amino acid sequence of the castor-Δ⁹18:0-ACP desaturase. Target codons corresponding to Met 114, Leu 118, Pro 179, and Gly 188 were each subjected to independent randomization. Because these residues are located adjacent to the substrate binding cavity, amino acid substitutions at these positions are highly likely to affect substrate specificity. These mutagenesis reactions yielded four populations, each one comprising a library of coding sequences with substitution mutations consisting of all 20 potential amino acids at the designated mutation site. Random mutagenesis was performed on sequences encoding castor-Δ⁹18:0-ACP desaturase by single gene DNA shuffling.

MH13(pACYC/LacAnFd) were transformed with the resulting libraries of mutated 18:0-ACP desaturase, under conditions appropriate for expression, and then selected for expression of a mutant with the ability to complement the unsaturated fatty acid auxotrophy, by growth in the absence of exogenous unsaturated fatty acid. To confer survival under the selective conditions, a mutant desaturase would necessarily have an altered substrate chain length specificity of 16, 14 or fewer carbons. The selection for site directed mutants was performed in either liquid media or on agar plates. The selection for randomly generated mutants was performed on agar plates. Growth in liquid media involved several rounds of dilution and re-growth to enrich for mutations that resulted in the best complementation. In a variation on the site directed mutagenesis, mutants were selected from a library encoding all 400 possible combinations of amino acids at position 179 and 114, two adjacent contact residues within the substrate binding channel. This was achieved by excising a restriction fragment from the open reading frame of the library encoding all possible amino acids at position 179 and inserting this fragment into the equivalent plasmid population randomized for position 114. Using this method, mutant M114I-G188L was selected. The coding sequences of the selected desaturases were sequenced to identify the specific mutations which conferred complementation to the fatty acid auxotrophy. The substrate specificities of the identified mutants were determined by in vitro enzyme assays (Cahoon et al., *Proc Natl Acad Sci U S A 94:* 4872-4877 (1997)).

Table 1 lists mutants initially identified and the altered chain length substrate specificity conferred.

**Table 1. Mutations obtained by mutagenesis/selection numbers in parentheses represent the chain length specificity that is most enhanced with respect to the wt castor castor-D9 18:0-desaturase activity. Shown are also the fold change in specificity ratios where known. For instance, if the activity with respect to 16 carbon was increased by 10-fold, and the activity with respect to 18:0 was decreased by 5-fold, the "Fold change in specificity with respect to wt for 16:18 would be 50.**

| | | | Fold change in specificity with respect to wt | |
|---|---|---|---|---|
| Position | Directed | Random | 16:18 | 14:18 |
| Met 114 | Ile (16) | Ile (16) | 6 | |
| Met 114 | Phe (14)/Tyr (14) | | Phe 7 | 490 |
| Thr 117 | | Ile (16) | not determined | |
| Leu 118 | Phe (16)/Tyr (16) | Phe (16)/Met (16) | Tyr 130 | |
| Pro 179 | Ile (16) | Leu (14) | 20 | |
| Thr 181 | | Ile (16) | not determined | |
| Gly 188 | Leu (16) | | 740 | |
| Met114-Gly188 | M1141-G188L (16) | | 1410 | |

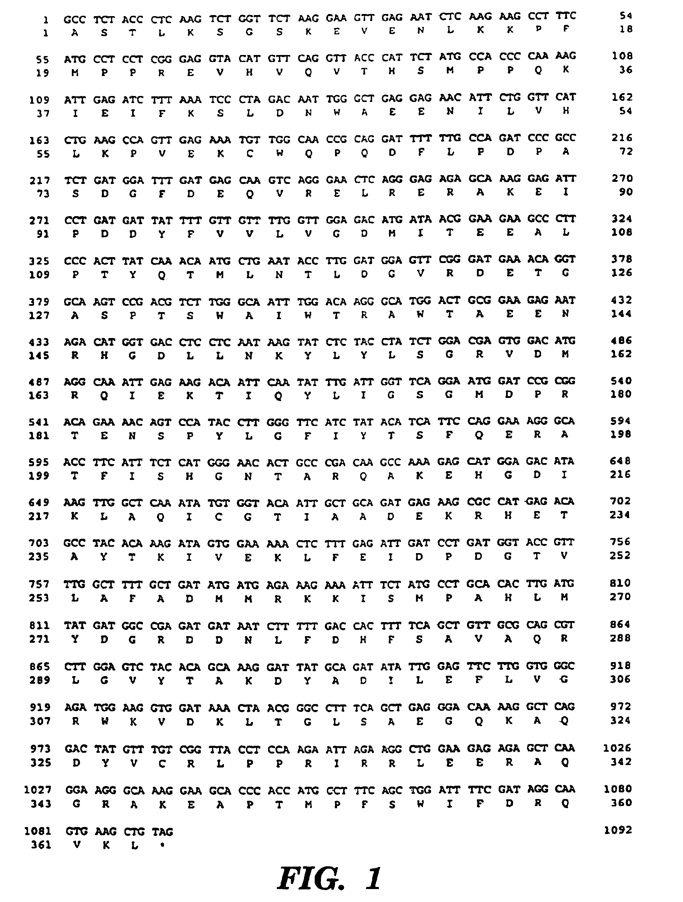

The designated amino acid positions above correspond to the mature castor enzyme as defined in Lindqvist et al., *EMBO J 15*: 4081-4092 (1996), the sequence of which is listed in Figure 1.

While the use of structure-guided (or directed) mutagenesis was effective for the identification of seven mutants with substrate specificities of 16 or fewer carbon fatty acids, the method relies on the appropriate choice of target residues for mutagenesis. It is well documented that residues that affect substrate specificity fall into two broad classes, direct and indirect. Thus, random mutagenesis selection provides a bias-free method for the identification of changes that result in increased specificity for shorter acyl chains. Five amino acid positions were identified with the use of this method, three at sites that were targets for the structure-guided mutagenesis, and two new sites, T117 and T181, were identified.

The naturally occurring 16:0-ACP desaturases from Milkweed and Doxantha have very poor activities when assayed in vitro. (31 and 3 nM/min/mg, respectively). However, the selected mutant G188L has an activity of (175 nM/min/mg) much closer to that of the parental wild type castor-Δ⁹ 18:0-ACP desaturase.

To test whether the altered enzymes identified in the selection assay would result in the accumulation of unusual fatty acids when expressed in plants, the G188L mutant was introduced into *Arabidopsis thaliana* (fab1 background) using a napin promoter to drive expression. The first generation of G188L transgenics (T1) produced seeds which contained approximately 10% of fatty acids modified by the introduced desaturase. Because T1 seeds are heterozygous it is anticipated the levels of desired fatty acids will increase in the homozygous T2 plants. These results suggest that mutants derived from castor-Δ⁹ 18:0-ACP desaturase may be useful for future metabolic engineering of oil crops.

### Materials and Methods for Section I

Cell lines. The *E. coli* unsaturated fatty acid auxotroph MH13 mutant of *E. coli* K12 (Henry, M.F., Ph.D. Thesis, University of Illinois, Urbana-Champaign (1992)) is a *fadR*::Tn5 mutant of cell line DC308 (Clark et al., *Biochemistry* 22: 5897-5902 (1983)) which was constructed by phage P1 transduction from strain RS3069 (Simons et al., *J. Bacteriol.* 142: 621-632 (1980)). MH13 requires exogenous unsaturated fatty acids at all growth temperatures due to a temperature-sensitive lesion in *fabA* and transposon disruption of *fadR.* An *Xba*I/*Eco*RI fragment from a pET9d expression plasmid containing the coding sequence of Anabaena vegetative ferredoxin (Fd) (Cheng et al., *Arch. Biochem. Biophys.* 316: 619-634 (1995)) was inserted into the corresponding sites of pLac3d to generate the plasmid pLacAnFd. pLac3d is analogous to pET3d except that the T7 RNA polymerase promoter has been replaced with the *lac*UV5 promoter of *E. coli* RNA polymerase as described previously (Cahoon et al., *J. Bacteriol.* 178: 936-939 (1996)). A *Bgl*II/*Hind*III fragment from pLacAnFd was then inserted into the *Bam*HI/*Hind*III sites of pACYC184. This construct (pACYC/LacAnFd) was then introduced into MH13 cells by electroporation.
Complementation Analysis/Selection. The *E*. *coli* MH13 strain harboring pACYC/LacAnFd was used as a host for expression of acyl-ACP desaturases. For these studies, the coding sequence of wild type and mutant mature acyl-ACP desaturases were inserted into pLac3d. Cells were transformed with the resulting plasmid constructs and were then grown on plates or in liquid broth containing Luria-Bertani (LB) media with ampicillin (100 µg/ml), chloramphenicol (35 µg/ml), and kanamycin (40 µg/ml) selection. For non-selective growth, plates were supplemented with the fatty acid oleic acid solubilized in Tergitol NP-40 (Sigma) with final concentrations of 250 µg/ml oleic acid and 2% (v/v) Tergitol. Liquid broth was supplemented with oleic acid (solubilized in Tergitol NP-40) at a final concentration of 100 µg/ml. Oleic acid was initially prepared as 1000x stock solution in ethanol and solubilized in melted Tergitol, prior to addition to the media. Media used to test for complementation contained IPTG at a concentration of 0.4 mM (to induce expression of acyl-ACP desaturase) with no added oleic acid.
Transformation. Transformation was conducted by electroporation using a 50 µl aliquot of competent MH13 cells harboring pACYC/LacAnFd and 0.1 to 0.5 µg of expression plasmid for a given acyl-ACP desaturase. Following electroporation, cells were resuspended in 500 µl of LB media and shaken (250 rpm) at 37°C for 45 min to 1 h. Cells were then plated on media as described above. Alternatively, a 75 µl aliquot of the transformed cells was added to 25-ml of LB media containing IPTG and antibiotics at concentrations described above. These cells were then maintained with shaking at 30° or 37°C.
   Electrocompetent MH13 (pACYC/LacAnFd) were prepared by growing a culture from a single colony in low-salt LB media (10 mg/ml Bacto tryptone, 5 mg/ml yeast extract, and 5 mg/ml sodium chloride) containing kanamycin (40 µg/ml) and chloramphenicol (35 µg/ml) and supplemented with oleic acid (100 µg/ml) and 2% Tergitol (v/v). Cells were prepared for transformation and electroporated as described in the BioRad protocol for high efficiency electro-transformation of *E. coli.*
   Mutagenesis. Two methods were used for mutagenesis. The first randomized a target residue at a specific location in the amino acid sequence of the castor-Δ⁹ 18:0-ACP desaturase. Four target residues were chosen: Met 114, Leu 118, Pro 179, and Gly 188. PCR was used to generate four populations of DNA. Each population consisted of sequences encoding castor-Δ⁹ 18:0-ACP desaturase with a randomized codon for residue 114, 118, 179, or 188. Each of the four populations was generated using PCR site directed mutagenesis to produce DNA products having equimolar proportions of each of the four nucleotides at each position of the target codon. For each of the four randomized products, an oligonucleotide primer was synthesized which hybridized to sequences adjacent to the target codon, and contained a randomized codon in place of the target codon sequences, the primer population containing equimolar proportions of each of the four nucleotides G, A, T, and C at the three positions within the replacement codon. This primer was used in conjunction with a primer homologous to the 5' terminus of the gene to amplify the gene segment between the two primer binding sites. A second overlapping fragment was then synthesized using PCR to amplify the remainder of the respective coding sequences of the four PCR reaction products. The fragments were then incorporated into larger gene fragments using overlap extension polymerase chain reaction (Ho et al., *Gene* 77: 51-59 (1989)). The gene fragments containing the randomized target codons were inserted into pLac3.

The second mutagenesis method introduced random mutations into the coding region sequence by digesting the castor-Δ⁹18:0-ACP desaturase coding region with DNase, and reassembling using PCR (W. P. Stemmer, *Proc Natl Acad Sci U S A 91:* 10747-10751 (1994)). The entire coding region was reinserted into pLac3 to make a library of pLac3-castor-Δ⁹ 18:0-ACP desaturase genes with random mutations throughout the coding region.

### SECTION II: COMPARISON OF FULL POSITIONAL RANDOMIZATION WITH OTHER METHODS

### Single Position Mutagenesis

Four amino acid positions in the castor Δ⁹-18:0-desaturase were identified by structural analysis (deductive reasoning by examination of a crystal structural model) as likely to participate in substrate specificity: 114, 118, 179, and 188. Independently, five positions were identified by random mutagenesis as likely to participate in substrate specificity: 114, 118, 179, 117, and 181. Together, this yielded a total of six positions identified as likely to participate in substrate specificity: 114, 117, 118, 179, 181, 188. To identify which of the 20 possible amino acids inserted at a target position would produce a mutant with the highest activity on 14 and 16 carbon substrates, libraries of mutants were generated by randomization of one of these positions at a time. Randomization resulted in insertion of 1 of all 20 possible amino acids at the target position. Six libraries were generated, one for each target position. These libraries were introduced into MH13 bacteria to select for mutants with enhanced substrate specificities of 14 or 16 carbon chain length. Complementation of MH13, the fatty acid auxotroph of *E*. *coli* requires that the desaturase have enhanced substrate specificity for 14 or 16 carbon chain length (or perhaps fewer). Recipient colonies which were able to grow under selective conditions were isolated. The mutant desaturase was purified from a crude lysate of a culture of the colony, and the specific activities for 14 and 16 carbon chain length substrates were determined for selected mutants by assay of the protein. For some mutants the specific activities of 18 carbon chain length substrates were determined.

Mutants produced from the two types of mutagenesis were identified and compared. Table 2 lists the most active mutants identified by each method. Table 3 compares the activities of mutants at position 117 and mutants at position 181, identified from both forms of mutagenesis. Mutants identified from the libraries of target position randomized mutants (full positional randomization) demonstrated the highest specific activities for 14 and 16 carbon substrates. Selection of mutants from a library produced by saturating random mutagenesis only once (L118F) produced the best mutant identified by the full positional randomization. Other selected mutants which resulted from random mutagenesis had catalytic rates inferior to the rates of mutants produced by full positional randomization. The reason for this is that random mutagenic process used could only generate between 4 and 7 amino acid substitutions per position. With respect to the six positions, at five of the positions, when offered all 19 substitutions by full positional randomization, a mutant enzyme with higher specific activity was obtained, and in the sixth case an equivalent activity was obtained. This demonstrated that the greater number of substitutions tested for a single position facilitated an increased probability of a higher increase in specific activity for a particular substrate.

**Table 2. Remodeling of the castor Δ⁹-18:0-desaturase using random point mutagenesis and full positional randomization.**

| | | **Random point mut.**^{**1**} | | | | **Full position randomization**^{**2**} | | |
|---|---|---|---|---|---|---|---|---|
| | | **Act**^{**3**} **nM/Min/mg** | | | | **Act nM/Min/mg** | | |
| **Site**^{**4**} | **Sub.**^{**5**} | **14** | **16** | **18** | **Sub.** | **14** | **16** | **18** |
| Met 114 | Ile | 3 | 22 | 260 | Ile | 3 | 22 | 260 |
| Met 114 | | | | | Phe/Tyr⁶ | 5 | 0.8 | 7 |
| Thr 117 | Ile | 0.7 | 5 | 35 | **Arg**⁶ | 3 | 30 | 332 |
| Leu 118 | Phe | 0.5 | 42 | 270 | Phe | 0.5 | 42 | 270 |
| Pro 179 | Leu | 15 | 22 | 206 | **Ile**^{**6**} | 18 | 78 | 270 |
| Thr 181 | Ile | 0.7 | 5 | 196 | **Phe**^{**6**} | 5 | 64 | 198 |
| Gly 188 | None Identified | | | | **Leu**⁶ | 11 | 173 | 19 |

| | | | | | | | | |
|---|---|---|---|---|---|---|---|---|
| Key: ¹ randomized point mutagenesis of the entire castor Δ⁹-18:0-desaturase. ²Full positional randomization using a primer encoding NNK to give 32-fold degeneracy with codons encoding all 20 amino acids. ³ Specific activity measured using ¹⁴C-argentation thin layer chromatography assay in conjunction with phosphor imaging quantitation. ⁴ Site of the amino acid position with respect to the mature castor open reading frame see following sheet for number definition. ⁵ Substitution, in standard three letter format. ⁶ Underlined, amino acid substitution that was not attainable by single point mutagenesis. | | | | | | | | |

**Table 3**

| | | | **Act**^{**1**}**nM/Min/mg** | | |
|---|---|---|---|---|---|
| **Site**^{**2**} | **Meth.** | **Sub.**^{**4**} | **14** | **16** | **18** |
| Wt | | None | 0.8 | 12 | 820 |
| Thr 117 | RPM³ | Ile | 0.7 | 5 | 35 |
| | FPR4 | Arg⁵ | 3 | 30 | 332 |
| | FPR4 | Val⁵ | 1 | 0.7 | 35 |
| | FPR4 | Lys⁵ | 3 | 2.6 | 93 |
| Thr 181 | RPM³ | Ile | 0.7 | 5 | 196 |
| | FPR⁴ | Phe⁵ | 5 | 64 | 198 |
| | FPR⁴ | Trp⁵ | 25 | 27 | 12 |
| | FPR⁴ | Leu⁵ | 2.4 | 42 | 181 |
| | FPR⁴ | Met⁵ | 3 | 15 | 479 |

| | | | | | |
|---|---|---|---|---|---|
| Key:¹ Specific activity measured using ¹⁴C-argentation thin layer chromatography assay in conjunction with phosphor imaging quantitation. ² Site of the amino acid position with respect to the mature castor open reading frame see following sheet for number definition. ³ RPM (randomized point mutagenesis of the entire castor Δ⁹-18:0-desaturase), ⁴ Full positional randomization (using a primer encoding NNK to give 32-fold degeneracy with codons encoding all 20 amino acids). ⁵ Underlined, amino acid substitution that was not attainable by single point mutagenesis. | | | | | |

### Combinatorial Full Positional Randomization

In an effort to produce a mutant desaturase protein which had enhanced activity for 14 and 16 carbon substrates, a library of mutants were generated by randomizing all six target sites, 114, 117, 118, 179, 179, and 188, simultaneously. This procedure is termed combinatorial full positional randomization. The library was introduced into MH13, which were then plated on selective media. A sample set of 19 colonies which grew on the selective media were picked. The plasmid DNA was isolated from each colony and used to re-transform the MH13 to confirm that the plasmids encoded a modified desaturase. Plasmids from each of the selected colonies were purified and the DNA sequence of each selected mutant encoded was determined.

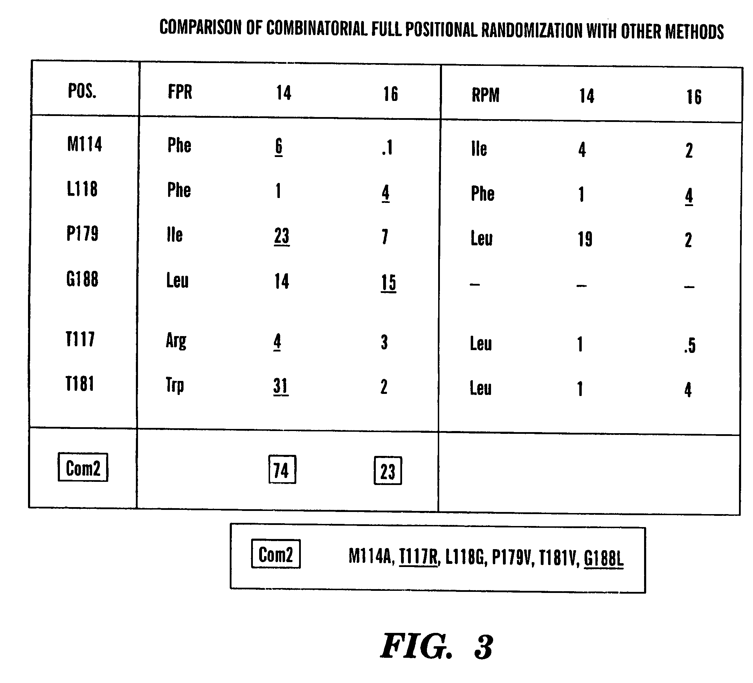

Conceptual translation of the DNA sequences indicated that all 19 mutants had distinct combinations of amino acids at the six target sites. All 19 mutant desaturase enzymes were produced and purified and subjected to in vitro enzyme assays. Figure 2 lists five of the mutants produced and their specific activities for the different substrates. The mutation having the highest specific activities for the tested substrates was com2. This mutant had far higher specific activity for 14 and 16 carbon substrates than did any of the single position mutants, exhibiting 74 and 23 times wild type activities for the respective substrates (Fig. 3). Two of the amino acid substitutions in com2, T117R, and G188L, were determined to be the optimal substitutions for those positions by the independent randomization studies described above. This correlation indicates that the increase in specificity of the com2 mutant is due to the substitutions at these two residues. Notably, neither of these changes were available via point mutagenesis. As indicated in Figure 2, five of the 19 combinatorial mutants analyzed contained these two specific mutations, T117R and G188L, but contained different substitutions at the other four positions. These five mutants exhibited various changes in specific activity for the two substrates. Thus substitutions at positions 114, 118, 179 and 181 had a profound effect on the influence of the changes at positions 117 and 188. Therefore, the combination of substitutions at the other four sites could either accentuate the positive effects that T117R and G188L had on activity, or alternatively, could block the effect. Increasing the number of possible combinations of amino acids at all positions identified as affecting the substrate specificity (e.g. when substituted individually), including amino acid substitutions which are sub-optimal for affecting the substrate specificity individually, yields mutants with optimal activities.

Indeed, the accommodation of mutations which produce positive changes without in turn causing negative changes is likely of great importance for obtaining optimal performance. One could, in a sense, look at amino acids as molecular shims in the structure, the more shims of different sizes and properties that can be used to modulate the structure, the higher the likelihood that any particular structure will have optimal activity. Thus the approach of identifying as many positions as possible which might affect a particular property of the protein, and then presenting as many combinations at each of those positions as possible, coupled with an appropriate screening process, will identify a mutant protein which has optimal activity. For the positions mutated in this example, point mutagenesis could result in a limited number of amino acid substitutions: 6 at M114; 5 at T117; 5 at L118; 6 at P179; 6 at T181; and 5 at G188. Thus the total combinatorial number would be 6³ x 5³ = 27,000 unique mutants. If all 20 combinations were permissible as in the combinatorial full positional randomization method, that number would rise to 19⁶= 47,045,881, or 1742-fold more combinations from which to select the optimal mutant for the particular trait. Since subtle changes can dramatically affect the activity of a protein, methods which result in more rather than fewer mutations of positions of amino acids shown to affect catalytic rates, will always produce equal or superior results to methods employing the more restrictive point mutagenesis.

### Materials and Methods For Section II are the same as those used for Section I, unless otherwise described below.

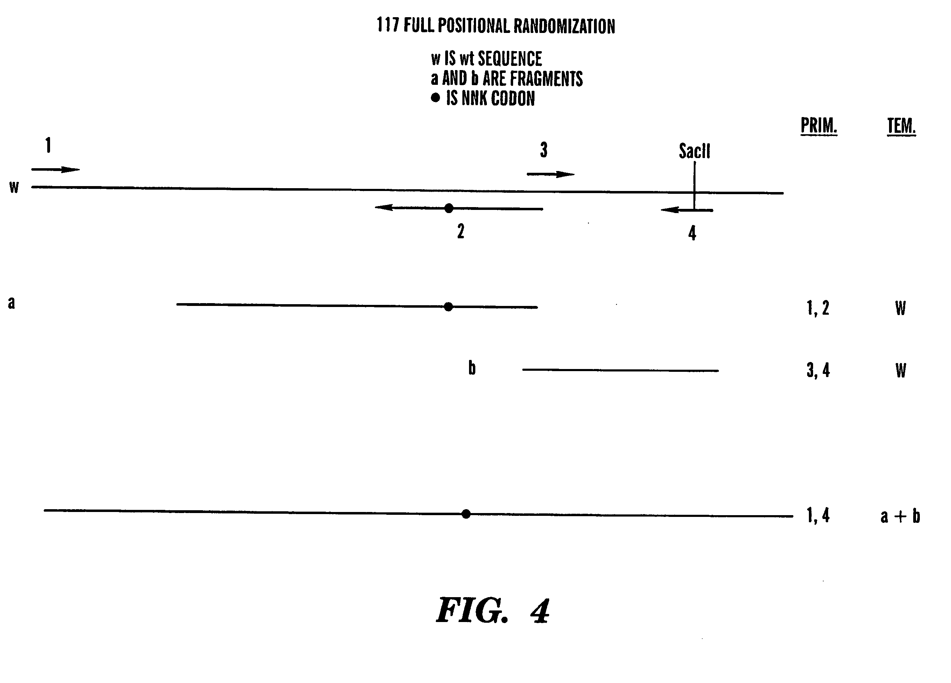

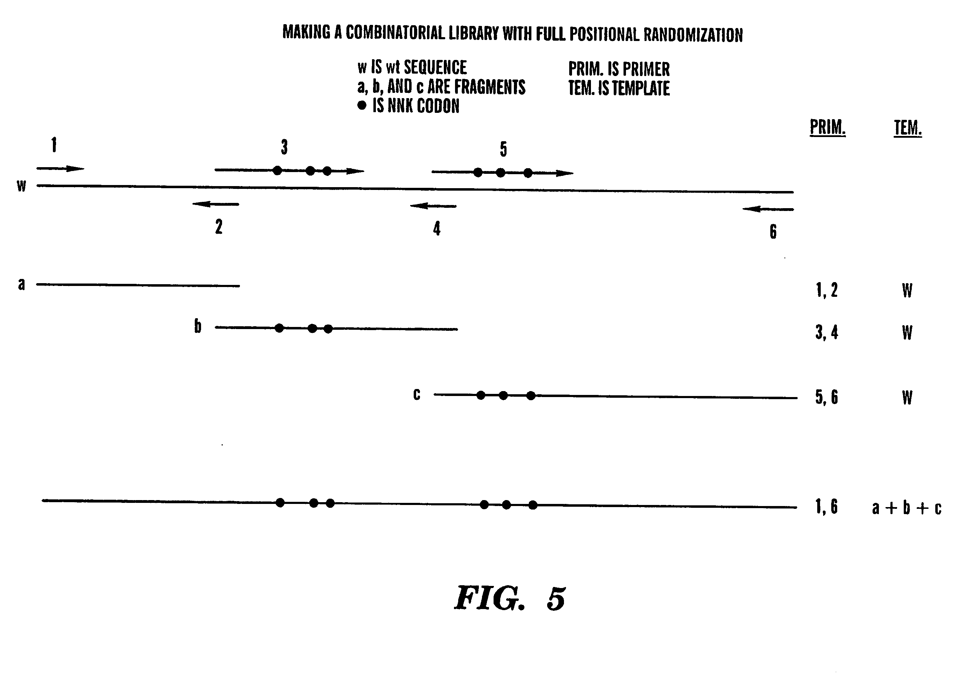

Full positional randomization. Castor-Δ⁹18:0-ACP desaturase was subjected to mutagenesis prior to introduction into the MH13 cells. PCR was used in site directed mutagenesis to randomize the three residues comprising a codon corresponding to a specified residue in the amino acid sequence of the castor-Δ⁹18:0-ACP desaturase. Target codons corresponding to Met 114, Thr 117, Leu 118, Pro 179, Thr 181 and Gly 188 were each subjected to independent randomization. Because these residues are located adjacent to the substrate-binding cavity, amino acid substitutions at these positions are considered highly likely to affect substrate specificity. These mutagenesis reactions yielded four populations, each one comprising a library of coding sequences with substitution mutations consisting of all 20 potential amino acids at the designated mutation site. An example for the introduction of all possible amino acid substitutions at position 117 by overlap extension PCR is diagrammed in Figure 4. Primers used were 1: GTGAGCGGATAACAATTTCACACAG-TCTAGAAAT, sequence flanking the unique XbaI site at the 5' end of the open reading frame; 2: CCAAATTGCCCAAGACGTCGGAC-TTGCACCTGTTTCATCCCGAACTCCATCCAAMNNATTCAGCATTGTTTG, the noncoding mutagenic oligonucleotide for position 117; 3: GAAACAGGTGCAAGTCCGACGTCTTGGGCAA, a non-mutagenic coding strand primer with overlap to the mutagenic 177 primer; 4: GTTTTCTGTCCGCGGATCCATTCCTG, a noncoding strand primer flanking the unique SacII site of the open reading frame. A PCR fragment was generated by overlap extension of fragments a and b using primers 1 and 4. This fragment was restricted by XbaI and SacII and introduced into the equivalent sites into pLac3 containing the wild type castor Δ⁹-18:0-desaturase. The other five positions of castor Δ⁹-18:0-desaturase were mutated independently in an equivalent fashion to methods used for position 117.
Random point mutagenesis. Random mutagenesis was performed on sequences encoding castor-Δ⁹ 18:0-ACP desaturase by single gene DNA shuffling (W. P. Stemmer, *Proc Natl Acad Sci U S A 91:* 10747-10751 (1994)). The open reading frame was first amplified by PCR using the primers: GTGAGCGGATAACAATTTCACACAGTCTAGAAAT which corresponds to the coding strand, and CACGAGGCCCTTTCGTCTTCAAGAATTCTC which corresponds to the noncoding strand. Approximately 50-200 bp from the wild type castor open reading frame was digested with DNaseI to make fragments at random positions within the open reading frame. The gene was then assembled by primerless PCR, followed by amplification of the full open reading frame using 5' and 3' specific primers. This method incorporated primarily point mutations at high frequency. The mutagenesis method used here was arbitrarily chosen, any method of point mutagenesis could have been used to produce equivalent results.
Combinatorial full positional randomization. For the combinatorial 6-site specific full positional randomization primers were engineered to contain NNK (where N refers to an equimolar mixture of GAT and C, and K refers to an equimolar mixture of G and T) for each of the six target codons. The full open reading frame was assembled and amplified by overlap extension PCR as shown in Figure 5. Primers corresponding to the diagram were 1: GTGAGCGGATAACAATTTCACACAGTCTAGAAAT, sequence flanking the unique XbaI site at the 5' end of the open reading frame; 2: TTGATAAGTGGGAAGGGCTTCTTCCGTT, non-mutagenic noncoding primer; 3: AACGGAAGAAGCCCTTCCCACTTATCAAACANNKCTGAATNNKNNKGATGGAGTTCG GGATGAAAC, mutagenic coding strand primer; 4: TCCATTCCTGAACCAATCAAATATTG non-mutagenic noncoding strand primer; 5: TTGATTGGTTCAGGAATGGATNNKCGGNNKGAAAACAGTCCATACCT-TNNKTTCATCTATACATCATTCC, mutagenic coding strand primer; 6: GCAAAAGCCAAAACGGTACCATCAGGATCA, noncoding non-mutagenic primer flanking the KpnI site. The three fragments were first amplified as shown in Figure 5. They were isolated and amplified by overlap-extension PCR as described above for full positional randomization of T117. The final fragment was restricted using XbaI and KpnI, and introduced into the equivalent sites in pLac3 containing the wild type castor Δ⁹-18:0-desaturase.
Selection of mutant desaturases with altered chain length specificity. To facilitate determination of the function of a plant acyl-ACP desaturase in *E*. *coli,* an expression vector containing the gene for plant-type ferredoxin, the redox partner of the plant desaturase, was transformed into the MH13 *E. coli* and maintained under selective pressure. These cells, MH13(pACYC/LacAnFd) were used in the following experiments. MH13(pACYC/LacAnFd) were transformed with the resulting libraries of mutated 18:0-ACP desaturase under conditions appropriate for expression. To achieve this, clones were restricted with either XbaI and KpnI, or XbaI and EcoRI, and introduced into the corresponding sites of plasmid pLac3 containing the mature castor Δ⁹-desaturase open reading frame. The plasmid pLac contains the Lac promoter which can be induced using the chemical inducer ispropyl β-thiogalactopyranoside (IPTG). Selection media lacking unsaturated fatty acids was used to identify mutants with the ability to complement the unsaturated fatty acid auxotrophy. To confer survival under the selective conditions, a mutant desaturase would necessarily have an altered substrate chain length specificity of 16, 14 or fewer carbons. The selection for site directed mutants was performed in either liquid media or on agar plates. The selection for randomly generated mutants was performed on agar plates. Growth in liquid media involved several rounds of dilution and re-growth to enrich for mutations that resulted in the best complementation. DNA for all mutants identified in this fashion was isolated and reintroduced into the mutant *E. coli* cell line, which was subjected to another round of selection to confirm the phenotype. The DNA of the selected desaturases were sequenced and translated conceptually to identify the specific mutations incurred.
Enzyme analyses. For determination of biochemical parameters of the desaturase mutants, the open reading frame was excised by restriction with XbaI and KpnI and ligated into the corresponding sites of the plasmid pLac3, which put the mature castor Δ⁹-desaturase open reading frame under the control of the T7 polymerase promoter. The plasmid was expressed in the cell line BL21DE3 Gold (Novagen) for expression. Cells were grown to 0.5 OD600, induced by addition of 0.4 mM IPTG and harvested after four hours. The desaturase enzyme was and extracted purified to near homogeneity (90%) by HPLC cation exchange chromatography using Poros 20CM media (Perseptive Biosystems). Purified desaturase was assayed using C1-¹⁴C acyl-ACP of appropriate chain lengths. Substrate and products were converted to methyl esters and analyzed by argentation thin layer chromatography and phoshor-imaging. Specific activities with the different substrates were calculated (Cahoon et al., *Proc Natl Acad Sci U S A 94*: 4872-4877 (1997)).

## Claims

1. A mutant castor Δ⁹ - 18 : 0 - ACP desaturase having each of the substitutions:
a) Ala for Met at residue 114;
b) Arg for Thr at residue 117;
c) Gly for Leu at residue 118;
d) Val for Pro at residue 179;
e) Val for Thr at residue 181; and
f) Leu for Gly at residue 188.

2. A DNA expression construct comprising, in expressible form, a nucleic acid sequence which encodes a mutant castor Δ⁹-18:0-ACP desaturase having each of the amino acid substitutions:
a) Ala for Met at residue 114;
b) Arg for Thr at residue 117;
c) Gly for Leu at residue 118;
d) Val for Pro at residue 179;
e) Val for Thr at residue 181; and
f) Leu for Gly at residue 188.

3. A cell transformed with a DNA expression construct comprising, in expressible form, a nucleic acid sequence which encodes a mutant castor Δ⁹ - 18 : 0 - ACP desaturase having each of the substitutions:
a) Ala for Met at residue 114;
b) Arg for Thr at residue 117;
c) Gly for Leu at residue 118;
d) Val for Pro at residue 179;
e) Val for Thr at residue 181; and
f) Leu for Gly at residue 188.

4. A transgenic plant expressing a nucleic acid sequence which encodes a mutant castor Δ⁹ - 18 : 0 - ACP desaturase having each of the amino acid substitutions:
a) Ala for Met at residue 114;
b) Arg for Thr at residue 117:
c) Gly for Leu at residue 118;
d) Val for Pro at residue 179;
e) Val for Thr at residue 181; and
f) Leu for Gly at residue 188.

5. The cell of claim 3 which is a prokaryotic cell, or a eukaryotic cell, for example a plant cell.

6. The transgenic plant of claim 4 which is *Arabidopsis thaliana.*

## Patentansprüche

1. Eine mutierte Castor Δ⁹ - 18 : 0 - ACP-Desaturase, die jede der Substitutionen
a) Ala gegen Met an Rest 114;
b) Arg gegen Thr an Rest 117;
c) Gly gegen Leu an Rest 118;
d) Val gegen Pro an Rest 179;
e) Val gegen Thr an Rest 181; und
f) Leu gegen Gly an Rest 188
aufweist.

2. Ein DNA-Expressionskonstrukt, das in exprimierbarer Form eine Nukleinsäuresequenz umfasst, die für eine mutierte Castor Δ⁹ - 18 : 0 - ACP-Desaturase kodiert, die jede der Aminosäure-Substitutionen
a) Ala gegen Met an Rest 114;
b) Arg gegen Thr an Rest 117;
c) Gly gegen Leu an Rest 118;
d) Val gegen Pro an Rest 179;
e) Val gegen Thr an Rest 181; und
f) Leu gegen Gly an Rest 188
aufweist.

3. Eine Zelle, die mit einem DNA-Expressionskonstrukt transformiert ist, das in exprimierbarer Form eine Nukleinsäuresequenz umfasst, die für eine mutierte Castor Δ⁹ - 18 : 0 - ACP-Desaturase kodiert, die jede der Substitutionen
a) Ala gegen Met an Rest 114;
b) Arg gegen Thr an Rest 117;
c) Gly gegen Leu an Rest 118;
d) Val gegen Pro an Rest 179;
e) Val gegen Thr an Rest 181; und
f) Leu gegen Gly an Rest 188
aufweist.

4. Transgene Pflanze, die eine Nukleinsäuresequenz exprimiert, die für eine mutierte Castor Δ⁹ - 18 : 0 - ACP-Desaturase kodiert, die jede der Aminosäure-Substitutionen
a) Ala gegen Met an Rest 114;
b) Arg gegen Thr an Rest 117;
c) Gly gegen Leu an Rest 118;
d) Val gegen Pro an Rest 179;
e) Val gegen Thr an Rest 181; und
f) Leu gegen Gly an Rest 188
aufweist.

5. Die Zelle gemäß Anspruch 3, die eine prokaryontische Zelle oder eine eukaryontische Zelle ist, zum Beispiel eine Pflanzenzelle.

6. Die transgene Pflanze gemäß Anspruch 4, die *Arabidopsis thaliana* ist.

## Revendications

1. Désaturase mutante de ricin Δ⁹-18:0-ACP (protéine portant un groupement acyle) comportant chacune des substitutions :
a) Ala au lieu de Met au résidu 114 ;
b) Arg au lieu de Thr au résidu 117 ;
c) Gly au lieu de Leu au résidu 118 ;
d) Val au lieu de Pro au résidu 179 ;
e) Val au lieu de Thr au résidu 181 ; et
f) Leu au lieu de Gly au résidu 188.

2. Construction d'ADN pour l'expression, comprenant, sous une forme exprimable, une séquence d'acide nucléique qui code pour une désaturase mutante de ricin Δ⁹-18:0-ACP comportant chacune des substitutions d'acide aminé:
a) Ala au lieu de Met au résidu 114 ;
b) Arg au lieu de Thr au résidu 117 ;
c) Gly au lieu de Leu au résidu 118 ;
d) Val au lieu de Pro au résidu 179 ;
e) Val au lieu de Thr au résidu 181 ; et
f) Leu au lieu de Gly au résidu 188.

3. Cellule transformée avec une construction d'ADN pour l'expression, comprenant, sous une forme exprimable, une séquence d'acide nucléique qui code pour une désaturase mutante de ricin Δ⁹-18:0-ACP comportant chacune des substitutions :
a) Ala au lieu de Met au résidu 114 ;
b) Arg au lieu de Thr au résidu 117 ;
c) Gly au lieu de Leu au résidu 118 ;
d) Val au lieu de Pro au résidu 179 ;
e) Val au lieu de Thr au résidu 181 ; et
f) Leu au lieu de Gly au résidu 188.

4. Plante transgénique exprimant une séquence d'acide nucléique qui code pour une désaturase mutante de ricin Δ⁹-18:0-ACP comportant chacune des substitutions d'acide aminé:
a) Ala au lieu de Met au résidu 114 ;
b) Arg au lieu de Thr au résidu 117 ;
c) Gly au lieu de Leu au résidu 118 ;
d) Val au lieu de Pro au résidu 179 ;
e) Val au lieu de Thr au résidu 181 ; et
f) Leu au lieu de Gly au résidu 188.

5. Cellule selon la revendication 3 qui est une cellule procaryote ou une cellule eucaryote, par exemple une cellule végétale.

6. Plante transgénique selon la revendication 4, qui est *Arabidopsis thaliana.*


### SEQUENCE LISTING

<110> Brookhaven Science Associates, LLC
<120> MUTANT FATTY ACID DESATURASE AND METHODS FOR DIRECTED MUTAGENESIS
<130> PCT filing of BNL2015W0
<140>
   <141>
<150> US 09/233,856
   <151> 1999-01-19
<150> US 09/328,550
   <151> 1999-06-09
<160> 14
<170> PatentIn Ver. 2.0
<210> 1
   <211> 363
   <212> PRT
   <213> Ricinus communis
<400> 1
   Ala Ser Thr Leu Lys Ser Gly Ser Lys Glu Val Glu Asn Leu Lys Lys
   1               5                   10                  15
   Pro Phe Met Pro Pro Arg Glu Val His Val Gln Val Thr His Ser Met
               20                  25                  30
   Pro Pro Gln Lys Ile Glu Ile Phe Lys Ser Leu Asp Asn Trp Ala Glu
           35                  40                  45
   Glu Asn Ile Leu Val His Leu Lys Pro Val Glu Lys Cys Trp Gln Pro
       50                  55                  60
   Gln Asp Phe Leu Pro Asp Pro Ala Ser Asp Gly Phe Asp Glu Gln Val
   65                  70                  75                  80
   Arg Glu Leu Arg Glu Arg Ala Lys Glu Ile Pro Asp Asp Tyr Phe Val
                   85                  90                  95
   Val Leu Val Gly Asp Met Ile Thr Glu Glu Ala Leu Pro Thr Tyr Gln
               100                 105                 110
   1
   Thr Met Leu Asn Thr Leu Asp Gly Val Arg Asp Glu Thr Gly Ala Ser
   115 120 125
   Pro Thr Ser Trp Ala Ile Trp Thr Arg Ala Trp Thr Ala Glu Glu Asn
   130 135 140
   Arg His Gly Asp Leu Leu Asn Lys Tyr Leu Tyr Leu Ser Gly Arg Val
   145 150 155 160
   Asp Met Arg Gln Ile Glu Lys Thr Ile Gln Tyr Leu Ile Gly Ser Gly
   165 170 175
   Met Asp Pro Arg Thr Glu Asn Ser Pro Tyr Leu Gly Phe Ile Tyr Thr
   180 185 190
   Ser Phe Gln Glu Arg Ala Thr Phe Ile Ser His Gly Asn Thr Ala Arg
   195 200 205
   Gln Ala Lys Glu His Gly Asp Ile Lys Leu Ala Gln Ile Cys Gly Thr
   210 215 220
   Ile Ala Ala Asp Glu Lys Arg His Glu Thr Ala Tyr Thr Lys Ile Val
   225 230 235 240
   Glu Lys Leu Phe Glu Ile Asp Pro Asp Gly Thr Val Leu Ala Phe Ala
   245 250 255
   Asp Met Met Arg Lys Lys Ile Ser Met Pro Ala His Leu Met Tyr Asp
   260 265 270
   Gly Arg Asp Asp Asn Leu Phe Asp His Phe Ser Ala Val Ala Gln Arg
   275 280 285
   Leu Gly Val Tyr Thr Ala Lys Asp Tyr Ala Asp Ile Leu Glu Phe Leu
   290 295 300
   Val Gly Arg Trp Lys Val Asp Lys Leu Thr Gly Leu Ser Ala Glu Gly
   305 310 315 320
   Gln Lys Ala Gln Asp Tyr Val Cys Arg Leu Pro Pro Arg Ile Arg Arg
   325 330 335
   Leu Glu Glu Arg Ala Gln Gly Arg Ala Lys Glu Ala Pro Thr Met Pro
   340 345 350
   Phe Ser Trp Ile Phe Asp Arg Gln Val Lys Leu
   355 360
<210> 2
   <211> 1092
   <212> DNA
   <213> Ricinus communis
<400> 2
   gcctctaccc tcaagtctgg ttctaaggaa gttgagaatc tcaagaagcc tttcatgcct 60
   cctcgggagg tacatgttca ggttacccat tctatgccac cccaaaagat tgagatcttt 120
   aaatccctag acaattgggc tgaggagaac attctggttc atctgaagcc agttgagaaa 180
   tgttggcaac cgcaggattt tttgccagat cccgcctctg atggatttga tgagcaagtc 240
   agggaactca gggagagagc aaaggagatt cctgatgatt attttgttgt tttggttgga 300
   gacatgataa cggaagaagc ccttcccact tatcaaacaa tgctgaatac cttggatgga 360
   gttcgggatg aaacaggtgc aagtccgacg tcttgggcaa tttggacaag ggcatggact 420
   gcggaagaga atagacatgg tgacctcctc aataagtatc tctacctatc tggacgagtg 480
   gacatgaggc aaattgagaa gacaattcaa tatttgattg gttcaggaat ggatccgcgg 540
   acagaaaaca gtccatacct tgggttcatc tatacatcat tccaggaaag ggcaaccttc 600
   atttctcatg ggaacactgc ccgacaagcc aaagagcatg gagacataaa gttggctcaa 660
   atatgtggta caattgctgc agatgagaag cgccatgaga cagcctacac aaagatagtg 720
   gaaaaactct ttgagattga tcctgatggt accgttttgg cttttgctga tatgatgaga 780
   aagaaaattt ctatgcctgc acacttgatg tatgatggcc gagatgataa tctttttgac 840
   cacttttcag ctgttgcgca gcgtcttgga gtctacacag caaaggatta tgcagatata 900
   ttggagttct tggtgggcag atggaaggtg gataaactaa cgggcctttc agctgaggga 960
   caaaaggctc aggactatgt ttgtcggtta cctccaagaa ttagaaggct ggaagagaga 1020
   gctcaaggaa gggcaaagga agcacccacc atgcctttca gctggatttt cgataggcaa 1080
   gtgaagctgt ag                                                    1092
<210> 3
   <211> 34
   <212> DNA
   <213> Artificial Sequence
<220>
   <223> Description of Artificial Sequence: PCR primer
<400> 3
   gtgagcggat aacaatttca cacagtctag aaat 34
<210> 4
   <211> 72
   <212> DNA
   <213> Artificial Sequence
<220>
   <223> Description of Artificial Sequence: PCR primer
<400> 4
   ccaaattgcc caagacgtcg gacttgcacc tgtttcatcc cgaactccat ccaamnnatt 60
   cagcattgtt tg                                                     72
<210> 5
   <211> 31
   <212> DNA
   <213> Artificial Sequence
<220>
   <223> Description of Artificial Sequence: PCR primer
<400> 5
   gaaacaggtg caagtccgac gtcttgggca a 31
<210> 6
   <211> 26
   <212> DNA
   <213> Artificial Sequence
<220>
   <223> Description of Artificial Sequence: PCR primer
<400> 6
   gttttctgtc cgcggatcca ttcctg 26
<210> 7
   <211> 34
   <212> DNA
   <213> Artificial Sequence
<220>
   <223> Description of Artificial Sequence: PCR primer
<400> 7
   gtgagcggat aacaatttca cacagtctag aaat 34
<210> 8
   <211> 30
   <212> DNA
   <213> Artificial Sequence
<220>
   <223> Description of Artificial Sequence: PCR primer
<400> 8
   cacgaggccc tttcgtcttc aagaattctc 30
<210> 9
   <211> 34
   <212> DNA
   <213> Artificial Sequence
<220>
   <223> Description of Artificial Sequence: PCR primer
<400> 9
   gtgagcggat aacaatttca cacagtctag aaat 34
<210> 10
   <211> 28
   <212> DNA
   <213> Artificial Sequence
<220>
   <223> Description of Artificial Sequence: PCR primer
<400> 10
   ttgataagtg ggaagggctt cttccgtt 28
<210> 11
   <211> 66 '
   <212> DNA
   <213> Artificial Sequence
<220>
   <223> Description of Artificial Sequence: PCR primer
<400> 11
   aacggaagaa gcccttccca cttatcaaac annkctgaat nnknnkgatg gagttcggga 60
   tgaaac 66
<210> 12
   <211> 26
   <212> DNA
   <213> Artificial Sequence
<220>
   <223> Description of Artificial Sequence: PCR primer
<400> 12
   tccattcctg aaccaatcaa atattg 26
<210> 13
   <211> 70
   <212> DNA
   <213> Artificial Sequence
<220>
   <223> Description of Artificial Sequence: PCR primer
<400> 13
   ttgattggtt caggaatgga tnnkcggnnk gaaaacagtc cataccttnn kttcatctat 60
   acatcattcc 70
<210> 14
   <211> 30
   <212> DNA
   <213> Artificial Sequence
<220>
   <223> Description of Artificial Sequence: PCR primer
<400> 14
   gcaaaagcca aaacggtacc atcaggatca 30